(19) **Europäisches Patentamt European Patent Office Office européen des brevets**

(11) **EP 2 344 022 B1**

(12) **EUROPEAN PATENT SPECIFICATION**

(45) Date of publication and mention
of the grant of the patent:
**30.08.2017 Bulletin 2017/35**

(51) Int Cl.:
**A61B 5/0452** (2006.01)     **A61N 1/365** (2006.01)
**A61N 1/37** (2006.01)

(21) Application number: **09788973.7**

(22) Date of filing: **22.07.2009**

(86) International application number:
**PCT/US2009/004242**

(87) International publication number:
**WO 2010/011301 (28.01.2010 Gazette 2010/04)**

(54) **MORPHOLOGY FEATURE EVALUATION IN IMPLANTABLE MEDICAL DEVICES**

BEWERTUNG EINES MORPHOLOGIE-MERKMALS IN IMPLANTIERBAREN MEDIZINISCHEN VORRICHTUNGEN

ÉVALUATION DE CARACTÉRISTIQUE DE MORPHOLOGIE DANS DES DISPOSITIFS MÉDICAUX IMPLANTABLES

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **22.07.2008 US 82732 P**

(43) Date of publication of application:
**20.07.2011 Bulletin 2011/29**

(73) Proprietor: **Cardiac Pacemakers, Inc.**
**St. Paul MN 55112-5798 (US)**

(72) Inventors:
• **DONG, Yanting**
**Shoreview**
**MN 55126 (US)**
• **BROOKE, M., Jason**
**Woodstock**
**MD 21163 (US)**

• **LI, Dan**
**Shoreview**
**MN 55126 (US)**
• **STALSBERG, Kevin, J.**
**White Bear Lake**
**MN 55110 (US)**

(74) Representative: **Pfenning, Meinig & Partner mbB**
**Patent- und Rechtsanwälte**
**Joachimsthaler Straße 10-12**
**10719 Berlin (DE)**

(56) References cited:
**WO-A-2004/012596      WO-A-2007/124270**
**WO-A2-2004/108212      US-A- 5 897 575**
**US-A1- 2007 239 220      US-A1- 2008 132 799**

**Description**

BACKGROUND

Implantable medical devices (IMDs) include devices designed to be implanted into a patient or subject. Some examples of these devices include cardiac function management (CFM) devices such as implantable pacemakers, implantable cardioverter defibrillators (ICDs), cardiac resynchronization therapy devices (CRTs), and devices that include a combination of such capabilities. The devices can be used to treat patients using electrical or other therapy, or to aid a physician or caregiver in patient diagnosis through internal monitoring of a patient's condition. The devices may include one or more electrodes in communication with one or more sense amplifiers to monitor electrical heart activity within a patient, and often include one or more sensors to monitor one or more other internal patient parameters. Other examples of implantable medical devices include implantable diagnostic devices, implantable drug delivery systems, or implantable devices with neural stimulation capability.

Additionally, some IMDs detect events by monitoring electrical heart activity signals. In CFM devices, these events can include heart chamber expansions or contractions. By monitoring cardiac signals indicative of expansions or contractions, IMDs can detect abnormally slow heart rate, or bradycardia. The monitoring can also be used to verify that electrical pacing therapy resulted in depolarization of a heart of a subject (e.g., used for sensing an evoked response).

Some IMDs detect abnormally rapid heart rate, or tachyarrhythmia. Tachyarrhythmia includes ventricular tachycardia (VT) and supraventricular tachycardia (SVT). Tachyarrhythmia also includes rapid and irregular heart rate, or fibrillation, including ventricular fibrillation (VF). When detected, ventricular tachyarrhythmia can be terminated using high-energy shock therapy applied with an ICD. It is important for IMDs to accurately classify sensed rhythms or arrhythmias.

US 2007/0239220 A1 discloses an apparatus comprising an implantable sensor and a processor for selecting ECG sensing vectors and applying the selected ECG signals to an ECG morphology detector. Means are provided for monitoring a signal quality metric for the selected sensing vectors.

US 2008/0132799 A1 discloses ECG morphology analysis using a plurality of features, wherein a correlation between different features is derived for data mining, and a confidence interval is determined for specific features, corresponding to the area within which one statistically expects to find the feature, thus enabling modification of the morphological identification algorithms and thus the quality of the automated detection of the features.

OVERVIEW

The invention is set out in the claims. In accordance with the invention, an apparatus includes an implantable sensor and a processor. The implantable sensor is configured to provide a plurality of physiologic sensor signals of a subject. The processor is communicatively coupled to the implantable sensor and includes a feature module and a detection module. The feature module is configured to identify at least one feature in at least one of the sensor signals and determine a measure of quality of the feature. The measure of quality includes at least one of a measure of dispersion of the feature, a measure of regularity of the feature, or a number of inflection points determined in the feature. The detection module is configured to perform a morphology analysis of a subsequent portion of at least one of the sensor signals using the feature when the measure of quality of the feature satisfies a quality measure threshold. The at least one feature includes a primary feature and a secondary feature different from the primary feature, and the detection module is adapted to use the primary feature in the morphology analysis when the measure of quality of the primary feature exceeds a primary quality measure threshold and remove the primary feature from the morphology analysis when the measure of quality of the primary feature fails to satisfy the primary quality measure threshold, and use the secondary feature in the morphology analysis when the measure of quality of the secondary feature satisfies a secondary quality measure threshold and remove the secondary feature from the morphology analysis when the measure of quality of the secondary feature fails to satisfy the secondary quality measure threshold. The measure of quality optionally includes the measure of dispersion. The detection module is optionally configured to select a particular feature for use in the morphology analysis when the measure of dispersion of the feature is less than a dispersion measure threshold. The measure of quality optionally includes the measure of regularity of the shape of the sensor signal segment comprising the feature. The detection module is optionally configured to select a particular feature for use in the morphology analysis when the measure of regularity of the shape of the sensor signal segment comprising the feature exceeds a regularity measure threshold.

The feature module is optionally configured to identify a peak value of at least one of the sensor signals. The measure of quality optionally includes the number of zero-value crossings in a gradient of the sensor signal segment comprising the feature. The gradient is obtained near a time of occurrence of the feature. The detection module is optionally configured to select a particular feature for use in the morphology analysis when the number of zero-value crossings is less than a threshold number.

The measure of quality optionally includes the number of inflection points determined in the feature. The detection module is optionally configured to select a particular feature for use in the morphology analysis when the number of inflection points is less than a threshold number.

The feature module is optionally configured to trend the measure of quality.

The implantable sensor optionally includes at least one of a cardiac signal sensing circuit, an intracardiac impedance sensor circuit, a transthoracic impedance sensor circuit, a blood pressure sensing circuit, a heart sound sensor circuit, an accelerometer, or a cardiac wall motion sensor circuit.

The feature module is optionally configured to identify the feature in at least one of the sensor signals using as the feature at least one of a maximum of the at least one sensor signal, a minimum of the at least one sensor signal, a slope of the at least one sensor signal, an area under a curve of a segment of the at least one sensor signal, a time when the at least one sensor signal reaches a specified amplitude, or an $N$th moment of the at least one sensor signal, wherein $N$ is a specified integer value.

The apparatus optionally includes a therapy circuit, communicatively coupled to the processor, configured to deliver an electrical therapy to the subject, and a cardiac signal sensing circuit, communicatively coupled to the processor, configured to provide an electrical cardiac signal representative of sensed heart activity of the subject. The detection module is optionally configured to perform the morphology analysis to determine at least one of a pacing vector, or an evoked response sensing vector.

The apparatus optionally includes a cardiac signal sensing circuit, communicatively coupled to the processor, configured to provide an electrical cardiac signal representative of sensed cardiac activity of the subject. The detection module is optionally configured to perform the morphology analysis to identify at least one of a detected heart rhythm, or a cardiac signal sensing vector.

The implantable sensor is optionally included in an implantable cardiac function management (CFM) device. The implantable CFM device includes a sampling circuit, communicatively coupled to the implantable sensor, configured to provide sampled sensor signals; and a communication circuit, communicatively coupled to the sampling circuit, configured to communicate information from at least one of the sampled sensor signals to an external device. The processor is included in the external device configured to communicate with the implantable CFM device.

The present disclosure further relates to examples of a method which do not form part of the claimed invention.

In example 1 of the method, the method includes receiving a plurality of implantably detected physiologic sensor signals, identifying a feature in at least one of the sensor signals using a medical device, determining a measure of quality of the feature, performing a morphology analysis of a subsequent portion of the at least one of the sensor signals using the using the feature when the measure of quality of the features satisfies a quality measure threshold, and providing an outcome of the morphology analysis to a user or process. The measure of quality includes at least one of a measure of dispersion of the feature, a measure of regularity of a shape of the sensor signal segment comprising the feature, a number of zero-value crossings in a gradient of the sensor signal segment comprising the feature, or a number of inflection points determined in the feature.

In example 2 of the method, determining the measure of quality of example 1 of the method optionally includes determining the measure of dispersion of the feature and the method includes performing the morphology analysis when the measure of dispersion of the feature is less than a dispersion measure threshold.

In example 3 of the method, determining the measure of quality of examples 1 and 2 of the method optionally includes determining the measure of regularity of the shape of the sensor signal comprising the feature, and the method includes performing the morphology analysis when the measure of regularity of the shape of the sensor signal exceeds a regularity measure threshold.

In example 4 of the method, identifying the feature in at least one of the sensor signals of examples 1-3 of the method optionally includes identifying a peak value of the at least one of the sensor signals, and the gradient of the sensor signal segment comprising the feature is determined near the feature. Determining the measure of quality optionally includes determining the number of zero-value crossings in the gradient, and using the feature in the morphology analysis optionally includes using the feature in the morphology analysis when the number of zero-crossings is less than a threshold number.

In example 5 of the method, determining the measure of quality of examples 1-4 of the method optionally includes determining the number of inflection points near the feature in the at least one sensor signal comprising the feature, and the detection module is optionally configured to use the feature in the morphology analysis when the number of inflection points is less than a threshold number.

In example 6 of the method, performing the morphology analysis of examples 1-5 of the method optionally includes performing the morphology analysis to identify a detected heart rhythm.

In example 7 of the method, the method of examples 1-6 of the method optionally includes, when the measure of quality of the feature ceases to satisfy the quality measure threshold, at least one of discontinuing using the feature in the morphology analysis, or changing a morphology threshold used in the morphology analysis.

In example 8 of the method, the method of examples 1-7 of the method optionally includes changing a vector configuration of the medical device in a manner so as to improve the measure of quality. In example 9 of the method, changing the vector configuration of the method of example 8 of the method optionally includes changing at least one of a cardiac signal sensing vector or a pacing vector.

In example 10 of the method, identifying the feature of examples 1-9 of the method optionally includes identifying a specified primary feature. The method of the examples optionally includes identifying a secondary feature different from the primary feature, and using the identified secondary feature to identify a detected heart rhythm when a measure of quality of the secondary feature satisfies a secondary quality measure threshold.

In example 11 of the method, identifying the feature of examples 1-10 of the method optionally comprises identifying at least one of: a maximum of the at least one sensor signal, a minimum of the at least one sensor signal, a slope of the at least one sensor signal, an area under a curve of a segment of the at least one sensor signal, a time when the at least one sensor signal reaches a specified amplitude, or an $N$th moment of the at least one sensor signal, wherein $N$ is a specified integer value.

In example 12 of the method, the medical device used in examples 1-11 of the method optionally comprises an external device. The method of the examples optionally includes: sampling the at least one sensor signal with an implantable medical device, communicating the sampled at least one sensor signal to the external device, and wherein performing the morphology analysis of the subsequent portion of the at least one of the sensor signals includes performing the morphology analysis using the external device.

This section is intended to provide an overview. It is not intended to provide an exclusive or exhaustive explanation of the invention. The detailed description is included to provide further information.

BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.

FIG. 1 is an illustration of portions of a system that uses an IMD.
FIG. 2 shows a graph of examples of cardiac signals.
FIG. 3 shows a graph of the peak points for the positive and negative peak values of the cardiac signals of FIG. 2.
FIG. 4 shows a graph of other examples of cardiac signals representative of evoked response.
FIG. 5 shows a graph of the peak points of the cardiac signals of FIG. 4.
FIG. 6 shows an example of a method of using a medical device to identifying features in sensor signals.
FIG. 7 is a graph showing an example of a sensor signal and its determined signal gradient.
FIG. 8 is a graph showing an expanded view of the signal gradient in FIG. 7.
FIG. 9 is a graph showing another example of a sensor signal and its determined gradient.
FIG. 10 is a graph showing an expanded view of the signal gradient in FIG. 9.
FIG. 11 is a graph of another example of a sensor signal.
FIG. 12 is a block diagram of portions of an example of a device to evaluate features in sensed physiologic signals.
FIG. 13 is a graph of another example of a sensor signal.
FIG. 14 shows another example of a method of identifying features in sensed electrical signals using a medical device.
FIG. 15 is a block diagram of portions of another example of a system to evaluate features in sensed physiologic signals.

DETAILED DESCRIPTION

An implantable medical device (IMD) may include one or more of the features or structures described herein. For example, a cardiac monitor or a cardiac stimulator may be implemented to include one or more of the advantageous features or processes described below. It is intended that such a monitor, stimulator, or other implantable or partially implantable device need not include all of the features described herein, but may be implemented to include selected features that provide for unique structures or functionality. Such a device may be implemented to provide a variety of therapeutic or diagnostic functions.

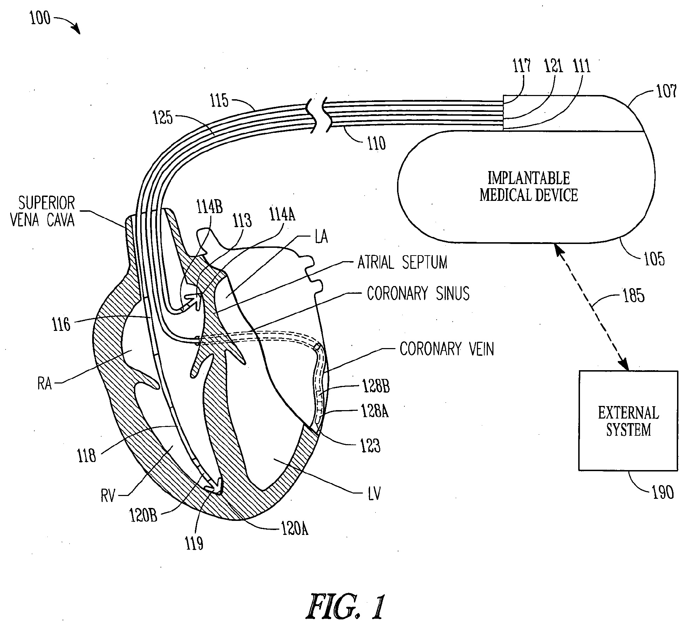

FIG. 1 is an illustration of portions of a system 100 that uses an IMD 105. Examples of IMD 105 include, without limitation, a pacemaker, a cardioverter, a defibrillator, a cardiac resynchronization therapy (CRT) device, and other cardiac monitoring and therapy delivery devices, including cardiac devices that include or work in coordination with one or more neuro-stimulating devices, drugs, drug delivery systems, or other therapies. As one example, the system 100 shown is used to treat a cardiac arrhythmia. The IMD 105 typically includes an electronics unit coupled by one or more cardiac leads 110, 115, 125, to a heart of a patient or subject. The electronics unit of the IMD 105 typically includes components that are enclosed in a hermetically-sealed canister or "can." The system 100 also typically includes an IMD programmer or other external system 190 that communicates one or more wireless signals 185 with the IMD 105, such as by using radio frequency (RF) or by one or more other telemetry methods.

The example shown includes right atrial (RA) lead 110 having a proximal end 111 and a distal end 113. The proximal end 111 is coupled to a header connector 107 of the IMD 105. The distal end 113 is configured for placement in the RA in or near the atrial septum. The RA lead 110 may include a pair of bipolar electrodes, such as an RA tip electrode 114A and an RA ring electrode 114B. The RA electrodes 114A and 114B are incorporated into the lead body at distal end 113 for placement in or near the RA, and are each electrically coupled to IMD 105 through a conductor extending within the lead body. The RA lead is shown placed in the atrial septum, but the RA lead may be placed in or near the atrial appendage, the atrial free wall, or elsewhere.

The example shown also includes a right ventricular (RV) lead 115 having a proximal end 117 and a distal end 119. The proximal end 117 is coupled to a header connector 107. The distal end 119 is configured for placement in the RV. The RV lead 115 may include one or more of a proximal defibrillation electrode 116, a distal defibrillation electrode 118, an RV tip electrode 120A, and an RV ring electrode 120B. The defibrillation electrode 116 is generally incorporated into the lead body such as in a location suitable for supraventricular placement in the RA and/or the superior vena cava. The defibrillation electrode 118 is incorporated into the lead body near the distal end 119 such as for placement in the RV. The RV electrodes 120A and 120B may form a bipolar electrode pair and are generally incorporated into the lead body at distal end 119. The electrodes 116, 118, 120A, and 120B are each electrically coupled to IMD 105, such as through one or more conductors extending within the lead body. The proximal defibrillation electrode 116, distal defibrillation electrode 118, or an electrode formed on the can of IMD 105 allow for delivery of cardioversion or defibrillation pulses to the heart.

The RV tip electrode 120A, RV ring electrode 120B, or an electrode formed on the can of IMD 105 allow for sensing an RV electrogram signal representative of RV depolarizations and delivering RV pacing pulses. In some examples, the IMD includes a sense amplifier circuit to provide amplification and/or filtering of the sensed signal. RA tip electrode 114A, RA ring electrode 114B, or an electrode formed on the can of IMD 105 allow for sensing an RA electrogram signal representative of RA depolarizations and allow for delivering RA pacing pulses. Sensing and pacing allows the IMD 105 to adjust timing of the heart chamber contractions. In some examples, the IMD 105 can adjust the timing of ventricular depolarizations with respect to the timing of atrial depolarizations by sensing electrical signals in the RA and pacing the RV at the desired atrial-ventricular (AV) delay time.

A left ventricular (LV) lead 125 can include a coronary pacing or sensing lead that includes an elongate lead body having a proximal end 121 and a distal end 123. The proximal end 121 is coupled to a header connector 107. A distal end 123 is configured for placement or insertion in the coronary vein. The LV lead 125 may include an LV ring or tip electrode 128A and an LV ring electrode 128B. The distal portion of the LV lead 125 is configured for placement in the coronary sinus and coronary vein such that the LV electrodes 128A and 128B are placed in the coronary vein. The LV electrodes 128A and 128B may form a bipolar electrode pair and are typically incorporated into the lead body at distal end 123. Each can be electrically coupled to IMD 105 such as through one or more conductors extending within the lead body. LV tip electrode 128A, LV ring electrode 128B, or an electrode formed on the can of the IMD 105 allow for sensing an LV electrogram signal representative of LV depolarizations and delivering LV pacing pulses.

An IMD may be configured with a variety of electrode arrangements, including transvenous, epicardial electrodes (i.e., intrathoracic electrodes), and/or subcutaneous, non-intrathoracic electrodes, including can, header, and indifferent electrodes, and subcutaneous array or lead electrodes (i.e., non-intrathoracic electrodes).

The electrodes and sense amplifier circuits may be included in an implantable cardiac signal sensing circuit. A cardiac signal sensing circuit obtains a sensed electrical cardiac signal associated with a depolarization of the patient's heart. Examples of cardiac signal sensing circuits include, among other things, a subcutaneous ECG sensing circuit, an intracardiac electro-gram (EGM) sensing circuit, and a wireless ECG sensing circuit. In a subcutaneous ECG sensing circuit, electrodes are implanted beneath the skin and the ECG signal obtained is referred to as subcutaneous ECG or far-field electrogram. In an intracardiac EGM circuit, at least one electrode is placed in or around the heart. A wireless ECG includes a plurality of electrodes to provide differential sensing of cardiac signals to approximate a surface ECG. Descriptions of wireless ECG systems are found in U.S. Patent Application Serial No. 10/795,126 by McCabe et al., entitled "Wireless ECG in Implantable Devices," filed on March 5, 2004. A morphology analysis is often used to detect a physiologic event in signals provided by sensors such as a cardiac signal sensing circuit. For example, discriminating between cardiac rhythms may include a combination of heart rate based tachyarrhythmia detection in combination with morphology based tachyarrhythmia classification. Heart rate based tachyarrhythmia detection may include a comparison of a measure of heart rate or rate interval to a corresponding threshold value programmed into the device. If the heart rate meets the criteria for tachyarrhythmia, the detection then proceeds to a morphology based rhythm classification.

A morphology-based analysis typically compares the morphological shape of a sensed cardiac depolarization to a template morphology, such as to classify a heart beat or heart rhythm. An approach to a combination heart rate and morphology based arrhythmia classification can be found in Kim et al., U.S. Patent Application Pub. No. 20070197928, "Rhythm Discrimination of Sudden Onset and One-to-One Tachyarrhythmia," filed February 17, 2006. In such a comparison, one or more features in a sensed signal are compared to a stored template signal. A correlation value can be determined (e.g., a feature correlation coefficient (FCC)) that can provide an indication of a degree of similarity between the shape of a depolarization being examined and the shape of the template to which it is compared. The FCC can be compared to a correlation threshold value to classify the rhythm as VT or SVT (e.g., ST, atrial fibrillation (AF), atrial flutter (AFL), or atrial tachyarrhythmia). However, identifying features for such an analysis may be difficult.

In another example, a morphology analysis may be used to detect pacing capture. A pace pulse must exceed a minimum energy value, or capture threshold, to produce a heart depolarization or contraction. Capture detection allows the cardiac rhythm management system to adjust the energy level of pace pulses to correspond to the optimum energy expenditure that reliably produces a depolarization. An analysis of the morphology of a sensed signal is used to detect capture.

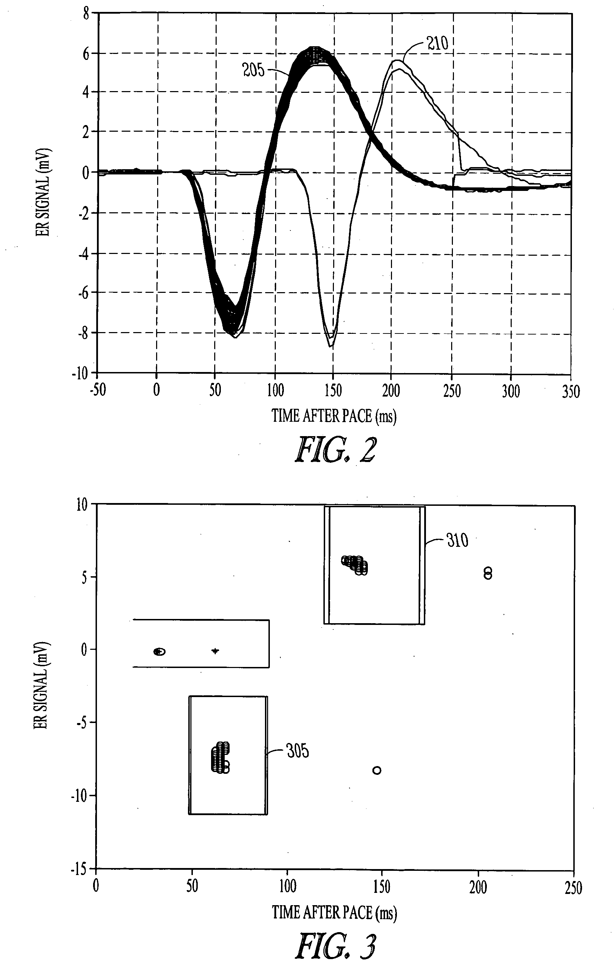

In certain examples, classification of a cardiac response to pacing involves sensing a cardiac response during a classification window. If a trigger feature of the cardiac signal is detected in the first classification window, a second cardiac response classification window is established. The cardiac signal is sensed in the second cardiac response classification window. The cardiac response to the pacing stimulation delivered to the chamber or combination of chambers is classified based on one or more characteristics of the cardiac signal. An approach to capture detection using sensed signal morphology is found in Myer et al., U.S. Patent Pub. No. 20050131477, "Cardiac Response Classification Using Retriggerable Classification Windows," filed December 12, 2003. In some examples, classification of a cardiac response to pacing involves obtaining a captured response template. One or more features of a cardiac signal sensed following a pacing stimulation are analyzed with respect to multiple classification windows. The classification windows may be defined based on the features of the captured response template. The cardiac response to pacing may be determined from a feature of the cardiac signal and the classification window in which the feature is detected. An approach to capture detection using a captured response template and sensed signal morphology is found in Kim et al., U.S. Patent No. 7,319,900, "Cardiac Response Classification Using Multiple Classification Windows," filed December 11, 2003. FIG. 2 shows a graph 200 of examples of cardiac signals. Most of the cardiac signals are included in the shaded region 205 with the exception of a few outliers 210. The cardiac signals in this example are representative of an evoked response of the subject. Morphology features of interest in such signals include the peak amplitude of the evoked response signal and the timing of the evoked response signal. The morphology feature may be used to verify the evoked response, to determine capture or non-capture during automatic capture verification, or for automatic threshold measurement applications.

FIG. 3 shows a graph 300 of the peak points for the positive and negative peak values of the cardiac signals. The graph 300 shows that the peak points exhibit a small amount of dispersion, or conversely exhibit good clustering of the peak values. In some examples, the measure of dispersion includes ranges of time and/or amplitude that provide boundaries for the peak points, or the measure includes a number of peak points that fall outside a predefined range as shown in windows 305, 310. In some examples, the measure of dispersion includes the standard deviation of time and/or amplitude of the peak points.

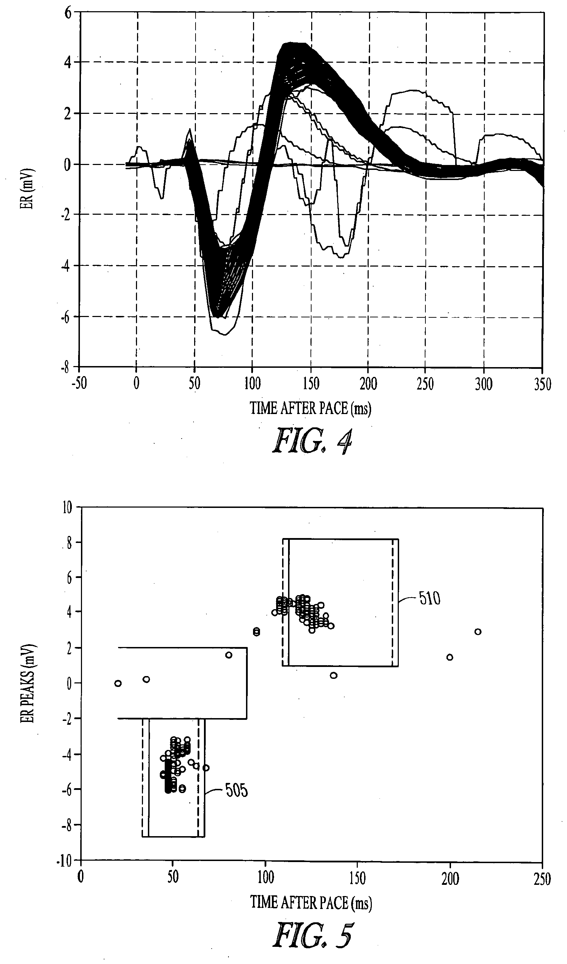

FIG. 4 shows a graph 400 of other examples of cardiac signals representative of evoked response. The cardiac signals in the graph are more dispersed in comparison to the signals in FIG. 2.

FIG. 5 shows a graph 500 of the peak points of the cardiac signals. The graph 500 shows that the peak points exhibit more dispersion. For example, a number of the peak points fall outside a predetermined range as shown in the windows 505 and 510.

Usually, the same feature or set of features is used for all patients in a morphology analysis, i.e., the set of features used by the analyzing medical device is static. Yet, FIGS. 4 through 5 show that a static set of features may prove difficult or unreliable. It is more desirable if the medical device is able to identify the best feature or features to use in its analysis (i.e., the medical device uses a set of features that are dynamic).

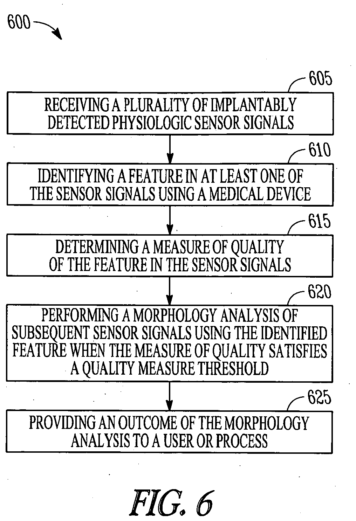

FIG. 6 shows an example of a method 600 of using a medical device to identifying features in sensor signals. At block 605, a plurality of implantably detected physiologic sensor signals is received at the medical device. At least one implantable sensor produces the signals, which may be electrical signals representative of a physiologic event of a subject. In some examples, the implantable sensor is a cardiac signal sensing circuit that provides an electrical signal representative of sensed heart activity. The sensor signals provided by the sensor may include cardiac signals which may be representative of a depolarization one or more chambers of the heart.

In some examples, the implantable sensor is a cardiac impedance sensor. A cardiac impedance sensor senses an electrical impedance signal between electrodes interposed in the heart. For example, in FIG. 1 a cardiac impedance sensor can sense intracardiac impedance between electrode 120B placed near the RV apex and electrode 116 placed in the right atrium. A predetermined excitation current is delivered between the electrodes and the impedance is determined from a voltage sensed between the electrodes. Systems and methods to measure intracardiac impedance are described in Citak et al., U.S. Pat. No. 4,773,401, entitled "Physiologic Control of Pacemaker Rate Using Pre-Ejection Interval as the Controlling Parameter," filed August 21, 1987. In FIG. 1, a transthoracic impedance of a subject can be measured between the ring electrode 120B and an electrode incorporated into the IMD can 105 or the IMD header 107. An approach to measuring transthoracic impedance is described in Hartley et al., U.S. Patent No. 6,076,015 "Rate Adaptive Cardiac Rhythm Management Device Using Transthoracic Impedance," filed February 27, 1998. In some examples, the implantable sensor is a cardiac pressure sensor. An implantable cardiac pressure sensor can be used to provide a sensor signal representative of heart chamber pressure. In an example, a pressure sensor may be implanted in a coronary vessel to determine left ventricle pressure by direct measurement of coronary vessel pressure. A description of systems and methods that use such an implantable pressure sensor is found in Salo et al., U.S. Patent No. 6,666,826, entitled "Method and Apparatus for Measuring Left Ventricular Pressure," filed January 4, 2002. Other cardiac pressure sensors examples include a right ventricle (RV) chamber pressure sensor, a pulmonary artery pressure sensor, and a left atrial chamber pressure sensor.

In some examples, the implantable sensor is a heart sound sensor circuit and the sensor signal is representative of mechanical vibrations of the heart. A description of systems and methods for monitoring heart sounds is found in U.S. Patent Application Serial No. 10/334,694, entitled "Method and Apparatus for Monitoring of Diastolic Hemodynamics," filed on December 30, 2002. In some examples, the implantable sensor includes an accelerometer and the sensor signal provided is representative of patient activity. An accelerometer can also be used to provide acceleration signals that are indicative of regional cardiac wall motion. One or more accelerometers can be incorporated into a portion of a lead positioned on or in the heart. The accelerometers detect the wall motion abnormality as an abrupt decrease in the amplitude of local cardiac accelerations. A description of systems and methods for sensing wall motion is found in U.S. Patent Application, Serial No. 11/135,985, entitled "Systems and Methods for Multi-Axis Cardiac Vibration Measurements," filed May 24, 2005. Returning to FIG. 6, at block 610, a feature is identified in the sensor signals. The feature may be a maximum of the sensor signals such as a positive peak value described previously. The feature may be a minimum of the sensor signals such as a negative peak value. Other features may be of interest besides peak values. In some examples, the feature is a time when the sensor signals reach a specified amplitude (e.g., a peak value or specified amplitude value). In some examples, the feature is a slope of a sensor signal or an area under a curve of a segment of the sensor signal. In some examples, the feature is an *N*th moment of the sensor signals; *N* being a specified integer value (e.g., the second moment, the third moment, or fourth moment). In certain examples, the *N*th moment is the *N*th

central moment $\mu_N$, where

$$\mu_N = E((X - \mu)^N).$$

At block 615, a measure of quality of the feature in the sensor signals is determined. In some examples, an IMD identifies the feature and determines the measure of quality. In some examples, one or more sensor signals are sampled by the IMD. The sampled signals are communicated wirelessly to an external medical device which identifies the feature and computes or calculates the measure of quality. This may be useful in offloading processing from the OVID if several features are to be identified and used. The quality measure is then compared to a quality measure threshold.

At block 620, a morphology analysis of a subsequent portion of the at least one of the sensor signals is performed using the identified feature according to the comparison of the measure to the threshold. If the quality measure satisfies the threshold, then a morphology analysis is performed using the feature. At block 625, the outcome of such a morphology analysis is provided to a user or process. The block functions may be performed by the IMD and the external device. For example, the IMD may identify the feature, determine the quality of the feature, and perform the morphology analysis.

The outcome of the morphology analysis may be used by the IMD in another analysis, used to change the behavior of the IMD, or communicated to an external device. Alternatively, sampled sensor signals may be sent to the external device for feature identification and evaluation, and the external device communicates the feature to the IMD to perform the morphology analysis; or the external device may perform the morphology analysis and communicate the results to the IMD. The external device may provide the result to a user.

The measure of quality is used to determine the usefulness of the feature in the analysis. In some examples, the measure of quality includes a measure of dispersion of the feature. The measure of dispersion may include the ranges of time and/or amplitude that provide boundaries (e.g., windows) for the peak points. In some examples, the measure of dispersion includes a number of peak points that fall outside a predefined range, or the measure may include the standard deviation of time and/or amplitude of the peak points. The identified feature is used in the morphology analysis when the dispersion measure is less than a dispersion measure threshold.

The feature may exhibit clustering that has more than one node or more than one cluster (e.g., two clusters that can each be bound within a window). In this case, one of the clusters may be used, or the feature may be discarded and not used at all.

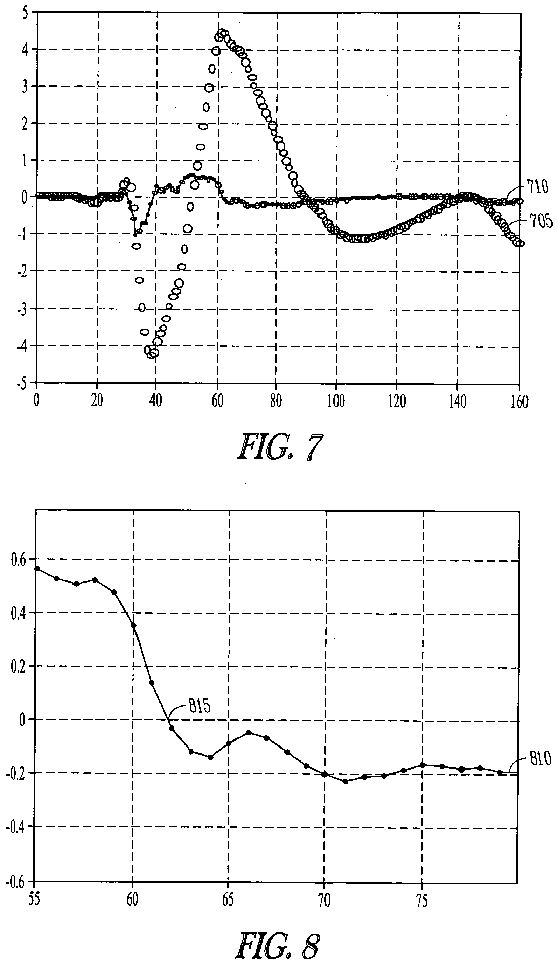

In some examples, the measure of quality includes a measure of regularity of the feature, or conversely a measure of variability of the feature. Examples of a measure of variability include, among other things, a variance of a fiducial representing the feature in the signal or a standard deviation of the fiducial. In some examples, the measure of quality includes a measure of regularity of the shape of the feature, such as the regularity of the width of the feature and/or the regularity of the amplitude of the feature. The identified feature is used in the morphology analysis when the measure of regularity of the shape of the feature exceeds a regularity measure threshold, or conversely when a measure of variability of the shape of the feature is less than a variability measure threshold. FIG. 7 is a graph showing an example of a sensor signal 705 and its calculated gradient 710, wherein the measure of quality is not in accordance with the invention. The gradient may be calculated by the medical device. In the example, the sensor signal 705 is representative of a cardiac depolarization and the identified feature is the peak value of the sensor signal. The measure of quality determined by the feature module includes the number of zero-value crossings in the gradient near a time of occurrence of the feature.

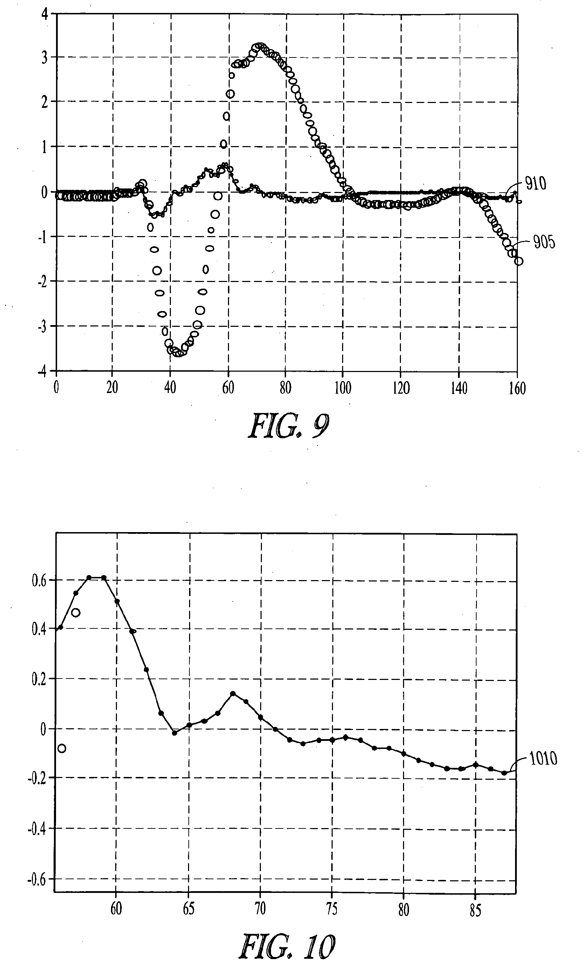

FIG. 8 is a graph showing an expanded view of the signal gradient 810, wherein the measure of quality is not in accordance with the invention. The graph shows that the signal gradient 810 only has one zero-crossing 815 near the time of occurrence of the feature. If the quality threshold is specified to be two zero-crossings, the number of zero-crossings satisfies the threshold number. FIG. 9 is a graph showing another example of a sensor signal 905 and its determined gradient 910. FIG. 10 is a graph showing an expanded view of the signal gradient 1010. The graph shows that the signal gradient has three zero-crossings near the time of occurrence of the feature and does not satisfy the threshold number.

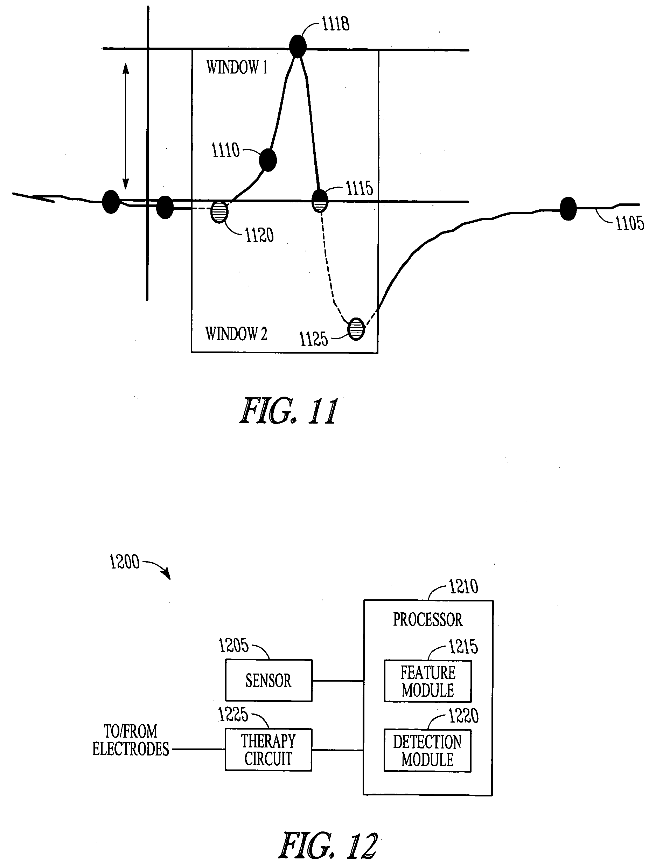

In some examples, the measure of quality includes a number of inflection points in the feature. FIG. 11 is a graph of another example of a sensor signal 1105. In the example, the sensor signal 1105 is representative of a cardiac depolarization. Possible feature points are indicated on the sensor signal.

Feature point 1118 and feature point 1125 are the positive and negative peaks respectively of the sensor signal 1105. Feature point 1110 and 1115 are determined based on points 1118 and 1125. If there are other inflection points around feature points 1118 or 1125, points 1118 and 1125 may move on a beat-by-beat basis, causing additional variation in feature points 1110 and 1115. This additional variation may result in errors in the morphology analysis. The inflection points can be determined from the zero-value crossing method mentioned above, or by a turning point method. In an example of a turning point method, curvature of a sampled sensor signal is computed on a sample-by-sample basis to form a curvature signal. Turns in the original sensor signal are reflected as excursions above and below a zero axis in the computed curvature signal. Descriptions of methods that determine inflection points using a turning point method are found in Sweeney et al., U.S. Patent Pub. No. 20040267143, "Signal Compression Based on Curvature Parameters," filed June 27, 2003. FIG. 12 is a block diagram of portions of an example of a device 1200 to evaluate features in sensed physiologic signals. The device 1200 includes one or more implantable sensors 1205. An implantable sensor provides a plurality of electrical sensor signals that are representative of a physiologic event of a subject. Examples of an implantable sensor 1205 include, among other things, an intrinsic cardiac signal sensing circuit, an intracardiac impedance sensor circuit, a transthoracic impedance sensor circuit, a blood pressure sensing circuit, a heart sound sensor circuit, an accelerometer, or a cardiac wall motion sensor circuit.

The device 1200 also includes a processor 1210. The processor 1210 is communicatively coupled to the implantable sensor 1205. Communicative coupling refers to devices arranged to communicate using electrical signals that influence the operation of the devices. In some examples, the devices are coupled directly. In some examples, the devices communicate electrical signals through intermediate devices, such as devices that include digital or analog circuits.

The processor 1210 may include a digital signal processor, application specific integrated circuit (ASIC), microprocessor, or other type of processor, interpreting or executing instructions in software or firmware. To provide the functions described herein, the processor 1210 includes modules. A module may include software, hardware, firmware or any combination thereof. For example, the module may include instructions in software executing on or interpreted by the processor 1210. Multiple functions may be performed by one or more modules.

The processor 1210 includes a feature module 1215. The feature module 1215 is configured to identify a feature in the sensor signals, and determine a measure of quality of the feature in the sensor signals. The measure of quality may include a measure of dispersion of the feature in the sensor signals, a measure of regularity of the feature in the sensor signals, and/or a number of inflection points determined in the feature in the sensor signals. The feature module 1215 is also configured to compare the measure of quality to a quality measure threshold.

The processor 1210 also includes a detection module 1220 configured to perform a morphology analysis of subsequent sensor signals using the identified feature according to a comparison of the measure of quality to a quality measure threshold.

According to some examples, the processor 1210 is configured to recursively evaluate the features identified in the sensor signals. In some examples, the feature module 1215 trends the measure of quality of the identified feature over time. If the measure of quality no longer satisfies the quality measure threshold (e.g., the measure of quality is below an acceptable threshold value, or exceeds an allowable threshold value), the detection may remove the feature from the morphology analysis or use the feature and change (e.g., reduce if appropriate) the threshold used in the morphology analysis. For example, the detection module 1220 may perform the morphology analysis to identify a detected heart rhythm. If the quality measure fails to satisfy the quality measure threshold, the detection module 1220 may use the identified feature, but reduce a FCC correlation threshold value used to classify the detected heart rhythm. In some examples, the feature module 1215 terminates the identifying when no features are identified or no features of acceptable quality are identified within a time duration.

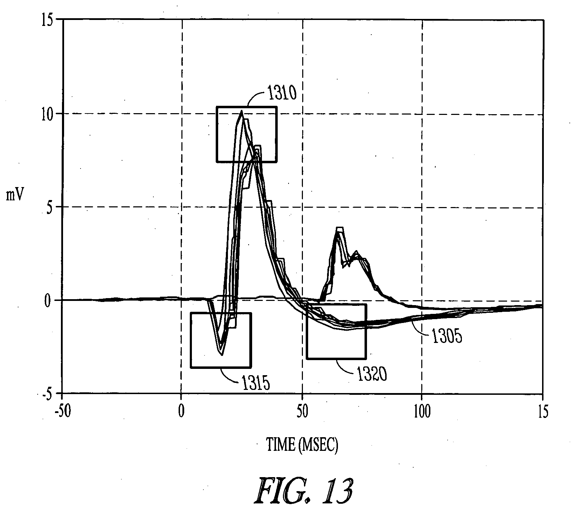

The feature module 1215 may identify more than one feature in the sensor signals. FIG. 13 is a graph of another example of a sensor signal 1305. The graph also shows timing-amplitude windows 1310, 1315, 1320 that the feature module 1215 uses to identify features. In accordance with the invention, the feature module 1215 identifies a specified primary feature and a secondary feature different from the primary feature. For example, the positive peak value in window 1310 of FIG. 13 may be the primary feature and the negative peak in window 1315 may be the secondary feature. The detection module 1220 uses the primary feature in the morphology analysis when the measure of quality of the primary feature exceeds the quality measure threshold, and uses the identified secondary feature to identify the detected heart rhythm when a measure of quality of the secondary feature satisfies a secondary quality measure threshold. In some examples, the feature module trends the measure of quality of at least one of the primary and secondary features. The detection module 1220 adds the feature to the analysis when the trending indicates the measure of quality exceeds the threshold quality measure value.

Adding additional features may improve the morphology analysis preformed by the detection module 1220. For example, identifying the additional features may increase the confidence level that the detected rhythm matches the template. Also, the additional features may make the analysis less susceptible to false positives due to noise.

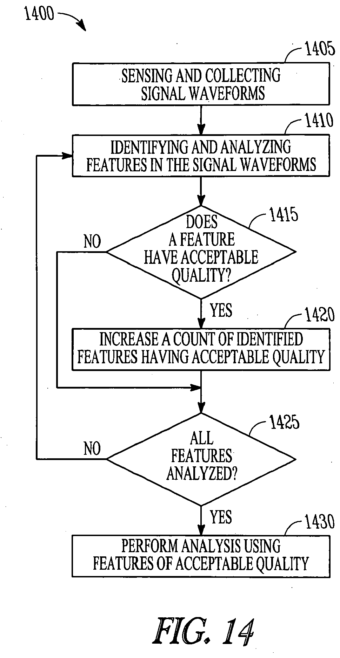

FIG. 14 shows another example of a method 1400 of identifying features in sensed electrical signals using a medical device. At block 1405, sensor signal waveforms are sensed and collected. In some examples, the waveforms are stored in a memory of the medical device. At block 1410, features are identified in the signal waveforms.

Table 1 represents examples of features identified by the medical device. Such a table may be stored in memory of the medical device. The medical device is identifying four features in three signal waveforms. The medical device may be programmed to search for those four features in the signal waveforms. The term "F21" refers to the first feature in the second signal waveform. Not all features maybe identified in the signals. The blank entry in the table represents feature number four not being identified in the second signal waveform.

Table 1

| Signal 1 | F11 | F12 | F13 | F14 |
|---|---|---|---|---|
| Signal 2 | F21 | F22 | F23 | |
| Signal 3 | F31 | F32 | F33 | F34 |

The medical device analyzes the identified features. At block 1415, it is determined if an identified feature has a measure of quality that satisfies a quality measure threshold. If so, at block 1420, the medical device may increase a count of features that have acceptable quality and are useful in an analysis (e.g., a morphology analysis) of subsequently sensed signal waveforms.

At block 1425, it is determined if all identified features in the signal wave forms have been identified. If not, the analysis continues at block 1410. If so, the table above may be reduced to the table below.

Table 2

| Signal 1 | | F12 | F13 | |
|---|---|---|---|---|
| Signal 2 | F21 | | F23 | |
| Signal 3 | | F32 | | F34 |

The table shows the identified features that were determined to have acceptable quality. At block 1430, the medical device performs the analysis. In accordance with the invention, features of unacceptable quality are removed from the analysis. Features of acceptable quality may be added to the analysis, or analysis parameters (e.g., measurement thresholds) may be adjusted.

Returning to FIG. 12, in some examples, the implantable sensor 1205 includes a cardiac signal sensing circuit communicatively coupled to the processor 1210. The detection module 1220 performs the morphology analysis to identify at least one of a detected heart rhythm as described above, or a cardiac signal sense vector. A vector refers to a combination of electrodes. Because the electrodes are used to sense electrical signals, sensing among different sets of electrodes, or vectors, often provides directional information regarding the propagation of cardiac signals. The device 1200 may include a switch matrix circuit to select different electrode combinations to use the best sensing vector.

In an illustrative example, the feature module 1215 identifies features deemed useful to detect evoked response of the heart to electrical pacing therapy, and the sensing vector is an evoked response sensing vector. Detecting evoked response provides verification that the pacing therapy resulted in depolarization of a heart of a subject. In some examples, the processor 1210 changes a sensing vector to improve the measure of quality in the feature by changing the electrode configuration used in the vector.

In some examples, the device 1200 includes a therapy circuit 1225 communicatively coupled to the processor 1210. The therapy circuit 1225 delivers an electrical therapy to the subject. The detection module 1220 is configured to perform the morphology analysis to determine a pacing vector. Choosing a different vector (e.g., different combination of electrodes) to deliver therapy often provides a different area to deliver the therapy, a different direction to provide the therapy, or a different timing relationship among the possible combinations.

In some examples, the implantable sensor 1205 and the processor 1210 are included in an implantable CFM device. In some examples, the functions may be performed by more than one medical device. In certain examples, the implantable sensor 1205 is included in an implantable CFM device and the processor 1210 is included in an external device.

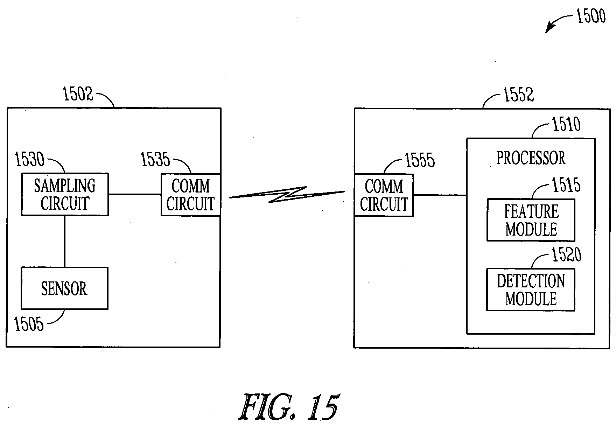

FIG. 15 is a block diagram of portions of another example of a system 1500 to evaluate features in sensed physiologic signals. One or more implantable sensors 1505 are included in an implantable CFM device 1502. The CFM device 1502 also includes a sampling circuit 1530 and a communication circuit 1535. The sampling circuit 1530 obtains digital samples (e.g., using an analog to digital converter) of the sensor signals thereby providing a sampled sensor signal. The communication circuit 1535 communicates the sampled sensor signals to another device.

The system 1500 also includes an external device 1552. An example of the external device 1552 includes a CFM device programmer. The external device 1552 includes a processor 1510 and a communication circuit 1555 to communicate with another device such as the implantable CFM device.

Sampled sensor signals are communicated to the external device 1552. The processor 1510 includes a feature module 1515 to identify a feature in the sampled sensor signals and to determine a measure of quality of the feature in the sampled sensor signals. In some examples, the identified feature is communicated to the CFM device. In certain examples, the identified feature is stored in the CFM device as part of a morphology template, and the CFM device performs the morphology analysis. In certain examples, the processor 1510 of the external device 1552 includes a detection module 1520 to perform the morphology analysis of subsequent sensor signals received from the CFM device 1502.

In some examples the system 1500 includes a repeater to relay communicated data or signals on to a second external device such as a server in an advanced patient management system. The processor is included in the second external device.

The above detailed description includes references to the accompanying drawings, which form a part of the detailed description. The drawings show, by way of illustration, specific embodiments in which the invention can be practiced.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In the appended claims, the terms "including" and "in which" are used as the plain-English equivalents of the respective terms "comprising" and "wherein." Also, in the following claims, the terms "including" and "comprising" are open-ended, that is, a system, device, article, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

Method examples described herein can be machine or computer-implemented at least in part. Some examples can include a computer-readable medium or machine-readable medium encoded with instructions operable to configure an electronic device to perform methods as described in the above examples. An implementation of such methods can include code, such as microcode, assembly language code, a higher-level language code, or the like. Such code can include computer readable instructions for performing various methods. The code can form portions of computer program products. Further, the code can be tangibly stored on one or more volatile or non-volatile computer-readable media during execution or at other times. These computer-readable media can include, but are not limited to, hard disks, removable magnetic disks, removable optical disks (e.g., compact disks and digital video disks), magnetic cassettes, memory cards or sticks, random access memories (RAM's), read only memories (ROM's), and the like.

The above description is intended to be illustrative, and not restrictive. The scope of the invention is defined by the appended claims.

## Claims

1. An apparatus comprising:

    an implantable sensor configured to provide a plurality of physiologic sensor signals of a subject;
    a processor, communicatively coupled to the implantable sensor, wherein the processor includes:

        a feature module, configured to:

            identify at least one feature in at least a first portion of at least one of the sensor signals, the at least one feature including a primary feature and a secondary feature different from the primary feature;

determine a measure of quality of the at least one feature, wherein the measure of quality includes at least one of:

a measure of dispersion of the feature;
a measure of regularity of the feature; or
a number of inflection points determined in the

feature; and

a detection module, configured to:

perform a morphology analysis of a subsequent portion of at least one of the sensor signals using the at least one feature when the measure of quality of the at least one feature satisfies a quality measure threshold;
wherein the detection module is adapted to use the primary feature in the morphology analysis when the measure of quality of the primary feature exceeds a primary quality measure threshold and remove the primary feature from the morphology analysis when the measure of quality of the primary feature fails to satisfy the primary quality measure threshold, and use the secondary feature in the morphology analysis when the measure of quality of the secondary feature satisfies a secondary quality measure threshold and remove the secondary feature from the morphology analysis when the measure of quality of the secondary feature fails to satisfy the secondary quality measure threshold.

2. The apparatus of claim 1, wherein the measure of quality includes the measure of dispersion, and wherein the detection module is configured to select a particular feature for use in the morphology analysis when the measure of dispersion of the feature is less than a dispersion measure threshold.

3. The apparatus of claim 2, wherein the measure of dispersion includes ranges of time that define boundaries used to identify clusters of the feature.

4. The apparatus of claim 1, wherein the measure of quality includes the measure of regularity of the shape of the sensor signal segment comprising the feature, and

wherein the detection module is configured to select a particular feature for use in the morphology analysis when the measure of regularity of the shape of the sensor signal segment comprising the feature exceeds a regularity measure threshold.

5. The apparatus of claim 1, wherein the measure of quality includes the number of inflection points determined in the feature, and
wherein the detection module is configured to select a particular feature for use in the morphology analysis when the number of inflection points is less than a threshold number.

6. The apparatus of any of claims 1-5, wherein the feature module is configured to trend the measure of quality.

7. The apparatus of any of claims 1-5, wherein the implantable sensor includes at least one of:

a cardiac signal sensing circuit;
an intracardiac impedance sensor circuit;
a transthoracic impedance sensor circuit;
a blood pressure sensing circuit;
a heart sound sensor circuit;
an accelerometer; or
a cardiac wall motion sensor circuit.

8. The apparatus of any of claims 1-5, wherein the feature module is configured to identify the feature in at least one of the sensor signals using as the feature at least one of:

a maximum of the at least one sensor signal;
a minimum of the at least one sensor signal;
a slope of the at least one sensor signal;
an area under a curve of a segment of the at least one sensor signal;
a time when the at least one sensor signal reaches a specified amplitude; or
an $N$th moment of the at least one sensor signal, wherein $N$ is a specified integer value.

9. The apparatus of any of claims 1-5, wherein the feature module is configured to identify the feature in at least one of the sensor signals using the $N$th moment of the at least one sensor signal, wherein the $N$th moment is the $N$th central moment $\mu_N$, wherein

$$\mu_N = E((X - \mu)^N).$$

10. The apparatus of any of claims 1-9, including:

a therapy circuit, communicatively coupled to the processor, configured to deliver an electrical therapy to the subject;

a cardiac signal sensing circuit, communicatively coupled to the processor, configured to provide an electrical cardiac signal representative of sensed heart activity of the subject, and wherein the detection module is configured to perform the morphology analysis to determine at least one of a pacing vector, or an evoked response sensing vector.

11. The apparatus of any of claims 1-10 including:

a cardiac signal sensing circuit, communicatively coupled to the processor, configured to provide an electrical cardiac signal representative of sensed cardiac activity of the subject, and wherein the detection module is configured to perform the morphology analysis to identify at least one of:

a detected heart rhythm; or
a cardiac signal sensing vector.

12. The apparatus of any of claims 1-11, wherein the implantable sensor is included in an implantable cardiac function management (CFM) device, the implantable CFM device including:

a sampling circuit, communicatively coupled to the implantable sensor, configured to provide sampled sensor signals; and
a communication circuit, communicatively coupled to the sampling circuit, configured to communicate information from at least one of the sampled sensor signals to an external device; and

wherein the processor is included in the external device configured to communicate with the implantable CFM device.

**Patentansprüche**

1. Vorrichtung, welche aufweist:

einen implantierbaren Sensor, der konfiguriert ist, mehrere physiologische Sensorsignale eines Subjekts zu liefern;
einen Prozessor, der kommunikativ mit dem implantierbaren Sensor gekoppelt ist, wobei der Prozessor enthält:

ein Merkmalsmodul, das konfiguriert ist zum:

Identifizieren zumindest eines Merkmals in zumindest einem ersten Teil zumindest eines der Sensorsignale, wobei das zumindest eine Merkmal ein primäres Merkmal und ein sekundäres Merkmal, das verschieden von dem primären Merkmal ist, enthält;

Bestimmen eines Maßes der Qualität des zumindest einen Merkmals, wobei das Maß der Qualität zumindest eines enthält von:

ein Maß der Streuung des Merkmals;
ein Maß der Regelmäßigkeit des Merkmals; oder
eine Anzahl von in dem Merkmal bestimmten Biegungspunkten; und

ein Erfassungsmodul, das konfiguriert ist zum:

Durchführen einer Morphologieanalyse eines nachfolgenden Teils zumindest eines der Sensorsignale unter Verwendung des zumindest einen Merkmals, wenn das Maß der Qualität des zumindest einen Merkmals einem Qualitätsmaß-Schwellenwert genügt; wobei das Erfassungsmodul ausgestaltet ist zur Verwendung des primären Merkmals bei der Morphologieanalyse, wenn das Maß der Qualität des primären Merkmals einen Primärqualitätsmaß-Schwellenwert überschreitet, und zum Entfernen des primären Merkmals aus der Morphologieanalyse, wenn das Maß der Qualität des primären Merkmals versagt, dem Primärqualitätsmaß-Schwellenwert zu genügen, und zum Verwenden des sekundären Merkmals in der Morphologieanalyse, wenn das Maß der Qualität des sekundären Merkmals dem Sekundärqualitätsmaß-Schwellenwert genügt, und zum Entfernen des sekundären Merkmals aus der Morphologieanalyse, wenn das Maß der Qualität des sekundären Merkmals versagt, dem Sekundärqualitätsmaß-Schwellenwert zu genügen.

2. Vorrichtung nach Anspruch 1, bei der das Maß der Qualität das Maß der Streuung enthält, wobei das Erfassungsmodul konfiguriert ist zum Auswählen eines speziellen Merkmals zur Verwendung bei der Morphologieanalyse, wenn das Maß der Streuung des Merkmals kleiner als ein Streuungsmaß-Schwellenwert ist.

3. Vorrichtung nach Anspruch 2, bei der das Maß der Streuung Zeitbereiche enthält, die zum Identifizieren von Clustern des Merkmals verwendete Grenzen definieren.

4. Vorrichtung nach Anspruch 1, bei der das Maß der Qualität das Maß der Regelmäßigkeit der Form des Sensorsignalsegments, das das Merkmal aufweist, enthält, und bei der das Erfassungsmodul konfiguriert ist zum Auswählen eines speziellen Merkmals für die Verwendung bei der Morphologieanalyse, wenn das Maß der Regelmäßigkeit der Form des Sensorsignalsegments, das das Merkmal aufweist, einen Regelmäßigkeitsmaß-Schwellenwert überschreitet.

5. Vorrichtung nach Anspruch 1, bei der das Maß der Qualität die Anzahl von in dem Merkmal bestimmten Biegungspunkten enthält, und bei der das Erfassungsmodul konfiguriert ist zum Auswählen eines speziellen Merkmals zur Verwendung bei der Morphologieanalyse, wenn die Anzahl von Biegungspunkten kleiner als eine Schwellenwertzahl ist.

6. Vorrichtung nach einem der Ansprüche 1- 5, bei der das Merkmalsmodul konfiguriert ist, den Trend des Maßes der Qualität zu setzen.

7. Vorrichtung nach einem der Ansprüche 1 - 5, bei der der implantierbare Sensor zumindest eine enthält von:

   eine Herzsignal-Erfassungsschaltung;
   eine Sensorschaltung für intrakardiale Impedanz;
   eine Sensorschaltung für transthorakale Impedanz;
   eine Blutdruck-Erfassungsschaltung;
   eine Herzton-Sensorschaltung;
   einen Beschleunigungsmesser; oder
   eine Herzwandbewegungs-Sensorschaltung.

8. Vorrichtung nach einem der Ansprüche 1 - 5, bei der das Merkmalsmodul konfiguriert ist zum Identifizieren des Merkmals in zumindest einem der Sensorsignale unter Verwendung von zumindest einem/einer von als das Merkmal:

   einem Maximum des zumindest einen Sensorsignals;
   einem Minimum des zumindest einen Sensorsignals;
   einer Neigung des zumindest einen Sensorsignals;
   einem Bereich unter einer Kurve eines Segments des zumindest einen Sensorsignals;
   einer Zeit, zu der das zumindest eine Sensorsignal eine bestimmte Amplitude erreicht; oder
   ein N-tes Moment des zumindest einen Sensorsignals, wobei N ein bestimmter ganzzahliger Wert ist.

9. Vorrichtung nach einem der Ansprüche 1 - 5, bei der das Merkmalsmodul konfiguriert ist zum Identifizieren des Merkmals in zumindest einem der Sensorsignale unter Verwendung des N-ten Moments des zumindest einen Sensorsignals, wobei das N-te Moment das N-te zentrale Moment $\mu_N$ ist, wobei

$$\mu_N = E((X - \mu)^N).$$

10. Vorrichtung nach einem der Ansprüche 1 - 9, enthaltend:

   eine Therapieschaltung, die kommunikativ mit dem Prozessor gekoppelt und konfiguriert ist zum Liefern einer elektrischen Therapie zu dem Subjekt;
   eine Herzsignal-Erfassungsschaltung, die kommunikativ mit dem Prozessor gekoppelt und konfiguriert ist zum Liefern eines elektrischen Herzsignals, das repräsentativ für die erfasste Herzaktivität des Subjekts ist, und wobei das Erfassungsmodul konfiguriert ist zum Durchführen der Morphologieanalyse, um zumindest einen von einem Schrittgabevektor oder einem Vektor einer erfassten hervorgerufenen Antwort zu bestimmen.

11. Vorrichtung nach einem der Ansprüche 1- 10, enthaltend:

   eine Herzsignal-Erfassungsschaltung, die kommunikativ mit dem Prozessor gekoppelt und konfiguriert ist, ein elektrisches Herzsignal zu liefern, das repräsentativ für die erfasste Herzaktivität des Subjekts ist, und wobei das Erfassungsmodul konfiguriert ist zum Durchführen der Morphologieanalyse für die Identifizierung von zumindest einem von:

   einem erfassten Herzrhythmus; oder
   einem Herzsignal-Erfassungsvektor.

12. Vorrichtung nach einem der Ansprüche 1 - 11, bei der der implantierbare Sensor in einer implantierbaren Herzfunktionsmanagement(CFM)-Vorrichtung enthalten ist, wobei die implantierbare CFM-Vorrichtung enthält:

   eine Abtastschaltung, die kommunikativ mit dem implantierbaren Sensor gekoppelt und konfiguriert ist, abgetastete Sensorsignale zu liefern; und
   eine Kommunikationsschaltung, die kommunikativ mit der Abtastschaltung gekoppelt und konfiguriert ist, Informationen von zumindest einem der abgetasteten Sensorsignale zu einer externen Vorrichtung zu übermitteln; und wobei der Prozessor in der externen Vorrichtung enthalten ist, die konfiguriert ist zum Kommunizieren mit der implantierbaren CFM-Vorrichtung.

**Revendications**

1. Appareil comprenant :

un capteur implantable qui est configuré de manière à fournir une pluralité de signaux de capteur physiologiques d'un sujet ;
un processeur, qui est couplé en communication au capteur implantable, dans lequel le processeur inclut :

un module de caractéristique, qui est configuré de manière à :

identifier au moins une caractéristique dans au moins une première partie d'au moins l'un des signaux de capteur, l'au moins une caractéristique incluant une caractéristique primaire et une caractéristique secondaire qui est différente de la caractéristique primaire ;
déterminer une mesure de qualité de l'au moins une caractéristique, dans lequel la mesure de qualité inclut au moins un élément informationnel pris parmi :

une mesure de dispersion de la caractéristique ;
une mesure de régularité de la caractéristique ; ou
un nombre de points d'inflexion déterminés dans la caractéristique ; et

un module de détection, qui est configuré de manière à :

réaliser une analyse de morphologie d'une partie suivante d'au moins l'un des signaux de capteur en utilisant l'au moins une caractéristique lorsque la mesure de qualité de l'au moins une caractéristique satisfait un seuil de mesure de qualité, dans lequel :

le module de détection est adapté de manière à utiliser la caractéristique primaire au niveau de l'analyse de morphologie lorsque la mesure de qualité de la caractéristique primaire excède un seuil de mesure de qualité primaire et de manière à enlever la caractéristique primaire de l'analyse de morphologie lorsque la mesure de qualité de la caractéristique primaire échoue à satisfaire le seuil de mesure de qualité primaire, et de manière à utiliser la caractéristique secondaire au niveau de l'analyse de morphologie lorsque la mesure de qualité de la caractéristique secondaire satisfait un seuil de mesure de qualité secondaire et de manière à enlever la caractéristique secondaire de l'analyse de morphologie lorsque la mesure de qualité de la caractéristique secondaire échoue à satisfaire le seuil de mesure de qualité secondaire.

2. Appareil selon la revendication 1, dans lequel la mesure de qualité inclut la mesure de dispersion, et dans lequel le module de détection est configuré de manière à sélectionner une caractéristique particulière pour une utilisation au niveau de l'analyse de morphologie lorsque la mesure de dispersion de la caractéristique est inférieure à un seuil de mesure de dispersion.

3. Appareil selon la revendication 2, dans lequel la mesure de dispersion inclut des plages de temps qui définissent des frontières qui sont utilisées de manière à identifier des groupes de la caractéristique.

4. Appareil selon la revendication 1, dans lequel la mesure de qualité inclut la mesure de régularité de la forme du segment de signal de capteur comprenant la caractéristique, et dans lequel le module de détection est configuré de manière à sélectionner une caractéristique particulière pour une utilisation au niveau de l'analyse de morphologie lorsque la mesure de régularité de la forme du segment de signal de capteur comprenant la caractéristique excède un seuil de mesure de régularité.

5. Appareil selon la revendication 1, dans lequel la mesure de qualité inclut le nombre de points d'inflexion déterminés dans la caractéristique, et dans lequel le module de détection est configuré de manière à sélectionner une caractéristique particulière pour une utilisation au niveau de l'analyse de morphologie lorsque le nombre de points d'inflexion est inférieur à un nombre de seuil.

**6.** Appareil selon l'une quelconque des revendications 1 à 5, dans lequel le module de caractéristique est configuré de manière à déterminer la tendance de la mesure de qualité.

**7.** Appareil selon l'une quelconque des revendications 1 à 5, dans lequel le capteur implantable inclut au moins un circuit pris parmi :

un circuit de détection de signal cardiaque ;
un circuit de capteur d'impédance intracardiaque ;
un circuit de capteur d'impédance thoracique ;
un circuit de détection de pression sanguine ;
un circuit de capteur de bruit du coeur ;
un accéléromètre ; ou
un circuit de capteur de déplacement de paroi cardiaque.

**8.** Appareil selon l'une quelconque des revendications 1 à 5, dans lequel le module de caractéristique est configuré de manière à identifier la caractéristique dans au moins l'un des signaux de capteur en utilisant, en tant que caractéristique, au moins un élément de caractérisation pris parmi :

un maximum de l'au moins un signal de capteur ;
un minimum de l'au moins un signal de capteur;
une pente de l'au moins un signal de capteur ;
une aire au-dessous d'une courbe d'un segment de l'au moins un signal de capteur ;
un temps/instant auquel l'au moins un signal de capteur attient une amplitude spécifiée ; ou
un N-ième moment de l'au moins un signal de capteur, où N est une valeur entière spécifiée.

**9.** Appareil selon l'une quelconque des revendications 1 à 5, dans lequel le module de caractéristique est configuré de manière à identifier la caractéristique dans au moins l'un des signaux de capteur en utilisant le N-ième moment de l'au moins un signal de capteur, où le N-ième moment est le N-ième moment central $\mu_N$, où :

$$\mu_N = E((X - \mu)^N).$$

**10.** Appareil selon l'une quelconque des revendications 1 à 9, incluant :

un circuit de thérapie, qui est couplé en communication au processeur, configuré de manière à délivrer une thérapie électrique au sujet ;
un circuit de détection de signal cardiaque, qui est couplé en communication au processeur, configuré de manière à fournir un signal cardiaque électrique qui est représentatif d'une activité de coeur détectée du sujet ; et dans lequel :

le module de détection est configuré de manière à réaliser l'analyse de morphologie de manière à déterminer au moins un vecteur pris parmi un vecteur de stimulation ou un vecteur de détection de réponse provoquée.

**11.** Appareil selon l'une quelconque des revendications 1 à 10, incluant :

un circuit de détection de signal cardiaque, qui est couplé en communication au processeur, configuré de manière à fournir un signal cardiaque électrique qui est représentatif d'une activité cardiaque détectée du sujet ; et dans lequel :

le module de détection est configuré de manière à réaliser l'analyse de morphologie afin d'identifier au moins un élément informationnel pris parmi :

un rythme de coeur détecté ; ou
un vecteur de détection de signal cardiaque.

**12.** Appareil selon l'une quelconque des revendications 1 à 11, dans lequel le capteur implantable est inclus dans un dispositif de gestion de fonction cardiaque (CFM) implantable, le dispositif de CFM implantable incluant :

un circuit d'échantillonnage, qui est couplé en communication au capteur implantable, configuré de manière à fournir des signaux de capteur échantillonnés ; et
un circuit de communication, qui est couplé en communication au circuit d'échantillonnage, configuré de manière à communiquer une information provenant d'au moins l'un des signaux de capteur échantillonnés à un dispositif externe ; et dans lequel :

le processeur est inclus dans le dispositif externe qui est configuré de manière à communiquer avec le dispositif de CFM implantable.

FIG. 1

FIG. 2

FIG. 3

**FIG. 4**

**FIG. 5**

600

605

RECEIVING A PLURALITY OF IMPLANTABLY
DETECTED PHYSIOLOGIC SENSOR SIGNALS

610

IDENTIFYING A FEATURE IN AT LEAST ONE OF
THE SENSOR SIGNALS USING A MEDICAL DEVICE

615

DETERMINING A MEASURE OF QUALITY
OF THE FEATURE IN THE SENSOR SIGNALS

620

PERFORMING A MORPHOLOGY ANALYSIS OF
SUBSEQUENT SENSOR SIGNALS USING THE IDENTIFIED
FEATURE WHEN THE MEASURE OF QUALITY SATISFIES
A QUALITY MEASURE THRESHOLD

625

PROVIDING AN OUTCOME OF THE MORPHOLOGY
ANALYSIS TO A USER OR PROCESS

*FIG. 6*

FIG. 7

FIG. 8

FIG. 9

FIG. 10

FIG. 11

FIG. 12

FIG. 13

1400 ⟍

1405

```
┌──────────────────────────────┐
│   SENSING AND COLLECTING     │
│      SIGNAL WAVEFORMS        │
└──────────────────────────────┘
```

1410

```
┌──────────────────────────────┐
│   IDENTIFYING AND ANALYZING  │
│ FEATURES IN THE SIGNAL WAVEFORMS │
└──────────────────────────────┘
```

1415

```
        DOES
      A FEATURE
   HAVE ACCEPTABLE
      QUALITY?
```

NO

YES

1420

```
┌──────────────────────────────────┐
│   INCREASE A COUNT OF IDENTIFIED  │
│ FEATURES HAVING ACCEPTABLE QUALITY│
└──────────────────────────────────┘
```

1425

```
        ALL
      FEATURES
      ANALYZED?
```

NO

YES

1430

```
┌──────────────────────────────┐
│    PERFORM ANALYSIS USING     │
│ FEATURES OF ACCEPTABLE QUALITY│
└──────────────────────────────┘
```

*FIG. 14*

*FIG. 15*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070239220 A1
- US 20080132799 A1
- US 79512604 A, McCabe
- US 20070197928 A, Kim
- US 20050131477 A, Myer
- US 7319900 B, Kim
- US 4773401 A, Citak
- US 6076015 A, Hartley
- US 6666826 B, Salo
- US 33469402 A
- US 13598505 A
- US 20040267143 A, Sweeney